# EUROPÄISCHE PATENTSCHRIFT

(11) **EP 0 737 211 B1**
(45) Veröffentlichungstag und Bekanntmachung des Hinweises auf die Patenterteilung: **04.06.1997**
(21) Anmeldenummer: 95938465.2
(22) Anmeldetag: 23.11.1995
(51) Int. Cl.: C08B 37/08, A61K 7/48, A61K 31/715

(54) **KATIONISCHE BIOPOLYMERE**
CATIONIC BIOPOLYMERS
BIOPOLYMERES CATIONIQUES

(30) Priorität: 02.12.1994 DE 4442987
(43) Veröffentlichungstag der Anmeldung: 16.10.1996
(73) Patentinhaber: Henkel Kommanditgesellschaft auf Aktien, 40191 Düsseldorf (DE); NORWEGIAN INSTITUTE OF FISHERIES AND AQUACULTURE LTD., 9002 Tromso (NO)
(72) Erfinder: WACHTER, Rolf, D-40595 Düsseldorf (DE); TESMANN, Holger, D-41363 Jüchen (DE); SVENNING, Ronald, N-9022 Krokelvdalen (NO); OLSEN, Ragnar, N-9015 Tromsö (NO); STENBERG, Even, N-9100 Kvalöysletta (NO)
(74) Vertreter: Wilk, Hans-Christof, Dr.
(86) Internationale Anmeldenummer: EP9504624
(87) Internationale Veröffentlichungsnummer: WO9616991

(56) Entgegenhaltungen:
- EP-A- 0 382 150
- WO-A-91/05808
- WO-A-96/23817
- FR-A- 2 701 029
- FR-A- 2 701 266
- US-A- 2 040 879
- MAKROMOLEKULARE CHEMIE, Bd. 177, 1976 DE, Seiten 3589-3600, TAKANORI SANNAN ET AL. 'Studies on chitin, 2. Effect of deacetylation on solubility.' in der Anmeldung erwähnt
- MAKROMOLEKULARE CHEMIE, Bd. 177, 1976 DE, Seiten 3589-3600, TAKANORI SANNAN ET AL. 'Studies on chitin, 2. Effect of deacetylation on solubility.'

## Beschreibung

### Gebiet der Erfindung

Die Erfindung betrifft kationische Biopolymere, die durch Demineralisierung, Deproteinierung, Decalcifizierung und Deacetylierung von frischen Krustentierschalen erhalten werden, ein Verfahren zu ihrer Herstellung sowie ihre Verwendung zur Herstellung von kosmetischen bzw. pharmazeutischen Mitteln.

### Stand der Technik

Chitosane stellen Biopolymere dar und werden zur Gruppe der Hydrokolloide gezählt. Chemisch betrachtet handelt es sich um partiell deacetylierte Chitine unterschiedlichen Molekulargewichtes, die den - idealisierten - Monomerbaustein (**I**) enthalten.

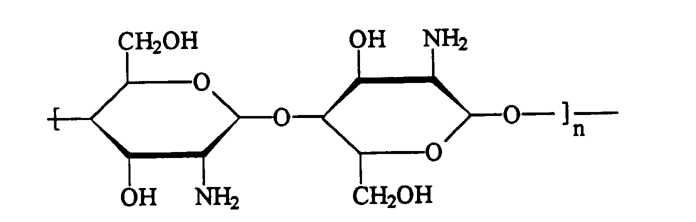

Im Gegensatz zu den meisten Hydrokolloiden, die im Bereich biologischer pH-Werte negativ geladen sind, stellen Chitosane unter diesen Bedingungen kationische Biopolymere dar. Die positiv geladenen Chitosane können mit entgegengesetzt geladenen Oberflächen in sowie pharmazeutischen Zubereitungen eingesetzt (vgl. **Ullmann's Encyclopedia of Industrial Chemistry, 5th Ed., Vol. A6, Weinheim, Verlag Chemie, 1986, S. 231-332)**. Übersichten zu diesem Thema sind auch beispielsweise von B.Gesslein et al. in **HAPPI 27**, 57 (1990), O.Skaugrud in **Drug Cosm. Ind. 148, 24 (1991)** und E.Onsoyen et al. in Seifen-**Öle-Fette-Wachse 117, 633 (1991)** erschienen.

Zur Herstellung der Chitosane geht man von Chitin, vorzugsweise den Schalenresten von Krustentieren aus, die als billige Rohstoffe in großen Mengen zur Verfügung stehen. Das Chitin wird dabei in einem Verfahren das erstmals von Hackmann et al. beschrieben worden ist, üblicherweise zunächst durch Zusatz von Basen deproteiniert, durch Zugabe von Mineralsäuren demineralisiert und schließlich durch Zugabe von starken Basen deacetyliert, wobei die Molekulargewichte über ein breites Spektrum verteilt sein können.

Aus **Makromol. Chem. 177, 3589 (1976)** ist ein Verfahren zur Herstellung eines Chitinabbauproduktes bekannt, bei dem man den Hackmann-Abbau dahingehend modifiziert, daß man die Krabbenschalen zunächst bei Raumtemperatur mit Salzsäure behandelt, anschließend bei 100°C über einen Zeitraum von 42 h mit Natronlauge deacetyliert, anschließend wieder bei Raumtemperatur mit Salzsäure und dann ebenfalls bei Raumtemperatur kurze Zeit mit Natronlauge nachbehandelt. Die Deacetylierung erfolgt bei diesem Verfahren im zweiten Schritt, die abschließende Behandlung mit Natronlauge dient hingegen nur zur ,,Feinjustierung" des Deacetylierungsgrades und findet daher bei Raumtemperatur statt. Dies führt zwar zu aschearmen Produkten mit hohem Deacetylierungsgrad und guter Löslichkeit in organischen Säuren, das Molekulargewicht ist jedoch sehr niedrig und die filmbildenden Eigenschaften unbefriedigend.

Gegenstand der Schrift **"Chitin, Chitosan, and Related Enzymes" (Ed. John P.Zikakis)**, **New York, Academic Press, 1984, S. XVII bis XXIV sowie S. 239 bis 255** sind u.a. ebenfalls Abbauprodukte des Chitins, die gemäß Tabelle I auf S. 248 ("Chitin D") zwar einen geringen Aschegehalt aufweisen, aber einen Deacetylierungsgrad besitzen, der mit nur 17,1 % äußerst niedrig liegt. Derartige Produkte sind in organischen Säuren jedoch völlig unlöslich.

Gegenstand der Europäischen Patentanmeldung **EP-A2 0 382 150** (Hoechst) ist ein Verfahren zur Herstellung von aktivierten Chitosanen, bei dem man Chitosan mit einer Säure zur Salzbildung und vorzugsweise anschließend mit einer Base umsetzt.

Aus der Intemationalen Patentanmeldung **WO 9105808** (Firextra Oy) ist ein Verfahren zur Herstellung von Chitosan bekannt, bei dem man Chitosan oder Chitin mit Alkalien deproteiniert, mit Säuren demineralisiert und mit Alkalien deacetyliert.

Aus der Französischen Patentanmeldung **FR-A 27 01 266** sind ebenfalls Abbauprodukte bekannt, die man erhält, indem man Chitin zunächst mit Salzsäure vorbehandelt und anschließend mit Natronlauge so weit als möglich deacetyliert. Es werden Produkte mit einem Deacetylierungsgrad von typischerweise 92 % erhalten, die sich durch einen sehr niedrigen.

Calciumcarbonatgehalt auszeichnen, in organischen Säuren gut löslich sind und dabei niedrigviskose Produkte ergeben. Von entscheidendem Nachteil ist jedoch wieder, daß infolge der drastischen Abbaubedingungen das Molekulargewicht sehr niedrig ist und auch hier wieder die filmbildenden Eigenschaften der Produkte unbefriedigend sind.

Zusammenfassend kann festgestellt werden, daß man die kationischen Biopolymere des Stands der Technik in zwei Gruppen einteilen kann: Zur ersten Gruppe von Produkten gehören diejenigen, die einen hohen Deacetylierungsgrad besitzen, in organischen Säuren löslich sind und dabei niedrigviskose Lösungen bilden, jedoch über keine ausreichenden filmbildenden Eigenschaften verfügen. In die zweite Gruppe gehören Produkte, die nur zu einem geringen Anteil deacetyliert worden sind, ein höheres Molekulargewicht und gute filmbildende Eigenschaften aufweisen, jedoch in organischen Säuren allenfalls schwerlöslich und daher nicht konfektionierbar sind.

Weiterhin weisen die Produkte des Stands der Technik eine Reihe von weiteren Nachteilen auf: Sie sind in der Regel als Folge des drastischen Abbaus stark verfärbt, geruchlich nicht akzeptabel und wenig lagerstabil, d.h. die Viskosität bleibt bei längerer Lagerzeit nicht erhalten, sondern nimmt ab. Weiterhin ist der Zusatz von Konservierungsmitteln erforderlich, wenn auch nicht gerne gesehen, da die Produkte anfällig gegenüber einer Kontamination durch Mikroorganismen sind.

Die komplexe Aufgabe der Erfindung hat demnach darin bestanden, neue kationische Biopolymere zur Verfügung zu stellen, die frei von den geschilderten Nachteilen sind, d.h. die gleichzeitig ein hohes Molekulargewicht aufweisen und dennoch in organischen Lösungen leicht und niedrigviskos löslich sind und trotz eines hohen Deacetylierungsgrades über ausgezeichnete filmbildende Eigenschaften verfügen.

### Beschreibung der Erfindung

Gegenstand der Erfindung sind neue kationische Biopolymere mit einem durchschnittlichen Molekulargewicht im Bereich von 800.000 bis 1.200.000 und vorzugsweise 900.000 bis 1.000.000 Dalton gemäß PLC einer Viskosität nach Brookfield (1 Gew.-%ig in Glycolsäure) unterhalb von 5000 mPas, einem Deacetylierungsgrad gemäß ¹H-NMR im Bereich von 80 bis 88 und vorzugsweise 82 bis 85 % und einem Aschegehalt von weniger als 0,3 Gew.-% und vorzugsweise weniger als 0,1 Gew.-%, die man erhält, indem man
(a) frische Krustentierschalen mit verdünnter wäßriger Mineralsäure behandelt,
(b) das resultierende demineralisierte erste Zwischenprodukt mit wäßriger Alkalihydroxidlösung behandelt,
(c) das resultierende geringfügig deproteinierte zweite Zwischenprodukt erneut mit verdünnter wäßriger Mineralsäure behandelt,
(d) gegebenenfalls das resultierende decalcifizierte dritte Zwischenprodukt bis auf einen Restwassergehalt von 5 bis 25 Gew.-% trocknet und
(e) schließlich mit konzentrierter wäßriger Alkalilauge deacetyliert,
wobei man die Schritte (a) und (c) bei einer Temperatur im Bereich von 15 bis 25°C und einem pH-Wert von 0,3 bis 0,7 und die Schritte (b) und (e) bei einer Temperatur im Bereich von 70 bis 110°C und einem pH-Wert von 12 bis 14 durchführt.

Überraschenderweise wurde gefunden, daß kationische Biopolymere, die man im wesentlichen durch Deacetylierung von seetierischem Chitin erhält, dann die eingangs gestellte Aufgabe erfüllen, wenn man das grundsätzlich bekannte Verfahren des wechselnden sauren und alkalischen Abbaus in der beschriebenen Weise unter genauer Einhaltung der Abfolge der einzelnen Schritte und der pH- und Temperaturbereiche durchführt. Es werden neue kationische Biopolymere erhalten, die trotz ihres hohen Molekulargewichtes in organischen Säuren leicht und rückstandsfrei löslich sind und gleichzeitig über überlegene filmbildende Eigenschaften verfügen. Zudem sind die Produkte hellfarbig, lagerstabil und ohne Zusatz von Konservierungsmitteln gegen Kontamination geschützt. Die Stoffe unterscheiden sich in ihrem Eigenschaftsprofil so signifikant von den bekannten Biopolymeren des Stands der Technik, daß sie als eigenständige neue Substanzen anzusehen sind.

Ein weiterer Gegenstand der Erfindung betrifft ein Verfahren zur Herstellung von kationischen Biopolymeren mit einem durchschnittlichen Molekulargewicht im Bereich von 800.000 bis 1.200.000 und vorzugsweise 900.000 bis 1.000.000 Dalton gemäß PLC einer Viskosität nach Brookfield (1 Gew.-%ig in Glycolsäure) unterhalb von 5000 mPas, einem Deacetylierungsgrad gemäß ¹H-NMR im Bereich von 80 bis 88 und vorzugsweise 82 bis 85 % und einem Aschegehalt von weniger als 0,3 und vorzugsweise weniger als 0,1 Gew.-%, die man erhält, indem man
(a) frische Krustentierschalen mit verdünnter wäßriger Mineralsäure behandelt,
(b) das resultierende demineralisierte erste Zwischenprodukt mit wäßriger Alkalihydroxidlösung behandelt,
(c) das resultierende geringfügig deproteinierte zweite Zwischenprodukt erneut mit verdünnter wäßriger Mineralsäure behandelt,
(d) gegebenenfalls das resultierende decalcifizierte dritte Zwischenprodukt bis auf einen Restwassergehalt von 5 bis 25 Gew.-% trocknet und
(e) schließlich mit konzentrierter wäßriger Alkalilauge deacetyliert,
wobei man die Schritte (a) und (c) bei einer Temperatur im Bereich von 15 bis 25°C und einem pH-Wert von 0,3 bis 0,7 und die Schritte (b) und (e) bei einer Temperatur im Bereich von 70 bis 110°C und einem pH-Wert von 12 bis 14 durchführt.

### Einsatzstoffe

Als Einsatzstoffe kommen Schalen von Krustentieren, vorzugsweise Krebs-, Krabben-, Shrimps- oder Krillschalen in Frage. Im Hinblick auf die Kontaminierung durch Endotoxine und die antimikrobielle Stabilisierung der Endprodukte hat es sich als vorteilhaft erwiesen, fangfrische Rohstoffe einzusetzen. Dies kann in der Praxis z.B. bedeuten, daß frisch gefangene Krabben noch an Bord des Schiffes von ihren Schalen befreit und diese bis zu ihrer Verarbeitung tiefgefroren werden. Es ist natürlich ebenso möglich, den gesamten Fang einzufrieren und an Land weiterzuverarbeiten.

### Demineralisierung

Die Demineralisierung stellt den besonders wichtigen ersten Schritt des erfindungsgemäßen Verfahrens dar, da auf diesem Wege ebenfalls Endotoxine entfernt, die Deproteinierung wesentlich erleichtert und schließlich die Grundlagen für die Herstellung von besonders aschearmen Biopolymeren gelegt werden. Die Demineralisierung wird durch Behandlung der Schalen mit wäßrigen Mineralsäuren, vorzugsweise verdünnter Salzsäure bei einer Temperatur im Bereich von 15 bis 25, vorzugsweise etwa 20°C und einem pH-Wert von 0,3 bis 0,7, vorzugsweise etwa 0,5 durchgeführt.

### Deproteinierung

Die Deproteinierung wird durch Behandeln der demineralisierten Zwischenprodukte mit wäßrigen Alkalihydroxidlösungen, vorzugsweise verdünnter 5 bis 25 Gew.-%iger Natriumhydroxidlösung erreicht. Vorzugsweise führt man diesen Schritt bei einer Temperatur im Bereich von 50 bis 110, insbesondere 90 bis 108°C und einem pH-Wert von 12 bis 14 durch.

### Decalcifizierung

Die Decalcifizierung gleicht in der Durchführung dem Demineralisierungsschritt. Auch hier wird das nunmehr deproteinierte Zwischenprodukt mit wäßrigen Mineralsäuren bei einer Temperatur im Bereich von 15 bis 25, vorzugsweise etwa 20°C und einem pH-Wert von 0,3 bis 0,7, vorzugsweise etwa 0,5 behandelt. Es hat sich als vorteilhaft erwiesen, das decalcifizierte Zwischenprodukt vor der nachfolgenden Deacetylierung bis auf einen Restwassergehalt von 5 bis 25 Gew.-% - bezogen auf das nicht entwässerte Produkt - zu trocknen.

### Deacetylierung

Die Deacetylierung wird unter Verwendung von konzentrierten Basen, beispielsweise konzentrierter 50 bis 70 Gew.-%iger Natron- oder Kalilauge wiederum bei einer Temperatur im Bereich von 70 bis 110, insbesondere 90 bis 108°C und einem pH-Wert von 12 bis 14 durchgeführt. Der Abbau erfolgt vorzugsweise in siedender Natronlauge und wird soweit durchgeführt, daß ein Biopolymer erhalten wird, das einen Gehalt von 0,1 bis 0,25, vorzugsweise 0,16 bis 0,2 Mol Acetamid pro Mol Monomerbaustein entsprechend einem Deacetylierungsgrad im Bereich von 80 bis 88 und vorzugsweise 82 bis 85 % resultiert. Der Deacetylierungsgrad kann im wesentlichen durch die Reaktionszeit eingestellt werden, die üblicherweise bei 1 bis 10 und vorzugsweise 2 bis 5 h liegt. Es hat sich ferner als vorteilhaft erwiesen, die Zwischenprodukte vor jedem neuen Verfahrensschritt neutral zu waschen.

### Gewerbliche Anwendbarkeit

Die neuen kationischen Biopolymere zeichnen sich dadurch aus, daß sie in wäßrigen Verdünnungen Gele einer außerordentlich hohen Viskosität bilden, Filme aufbauen und gegen mikrobiellen Befall weitgehend stabilisiert sind. Ein weiterer Gegenstand der Erfindung besteht demnach in der Verwendung der neuen Bipopolymere zur Herstellung von kosmetischen und/oder pharmazeutischen Mitteln, wie beispielsweise Haut- oder Haar- pflegemitteln, Hautrepairstoffen und Wundheilmitteln, in denen sie in Mengen von 0,01 bis 5, vorzugsweise 0,1 bis 1,5 Gew.-% - bezogen auf die Mittel - enthalten sein können.

### Hilfs- und Zusatzstoffe

Die Haut- und Haarpflegemittel mit einem Gehalt an den neuen kationischen Biopolymeren können mit ihnen kompatible **Tenside** enthalten.

Typische Beispiele für **anionische Tenside** sind Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretawide, Acyllactylate, Acyltartrate, Acylglutamate, Acylaspartate, Alkyloligoglucosidsulfate, Proteinfettsäurekondensate (insbesondere pflanzliche Produkte auf Weizenbasis) und Alkyl(ether) phosphate. Sofern die anionischen Tenside Polyglycoletherketten enthalten, können diese eine konventionelle, vorzugsweise jedoch eine eingeengte Homologenverteilung aufweisen.

Typische Beispiele für **nichtionische Tensid**e sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, Alk(en)yloligo-glykoside, Fettsäure-N-alkylglucamide, Proteinhydrolysate (insbesondere pflanzliche Produk-te auf Weizenbasis), Polyolfettsäureester, Zuckerester, Sorbitanester, Polysorbate und Amin-oxide. Sofern die nichtionischen Tenside Polyglycoletherketten enthalten, können diese eine konventionelle, vorzugsweise jedoch eine eingeengte Homologenverteilung aufweisen.

Typische Beispiele für **kationische Tenside** sind quartäre Ammoniumverbindungen und Esterquats, insbesondere quaternierte Fettsäuretrialkanolaminester-Salze.

Typische Beispiele für **amphotere bzw. zwitterionische Tenside** sind Alkylbetaine, Alkylamidobetaine, Aminopropionate, Aminoglycinate, Imidazoliniumbetaine und Sulfobetaine.

Bei den genannten Tensiden handelt es sich ausschließlich um bekannte Verbindungen. Hinsichtlich Struktur und Herstellung dieser Stoffe sei auf einschlägige Übersichtsarbeiten beispielsweise **J.Falbe (ed.), "Surfactants in Consumer Products", Springer Verlag, Berlin, 1987, S. 54-124 oder J.Falbe (ed.), "Katalysatoren, Tenside und Mineralöl-additive", Thieme Verlag, Stuttgart, 1978, S. 123-217** verwiesen. Besonders bevorzugt sind nichtionische, kationische, amphotere und/oder zwitterionische Tenside wie vorzugsweise Alkyl- und/oder Alkenyloligoglucoside, Fettsäure-N-alkylglucamide, Alkylamidobetaine, Proteinhydrolysate, quartäre Ammoniumverbindungen und Esterquats. Die Tenside können in den Mitteln in Mengen von 0,5 bis 15 Gew.-% - bezogen auf die Mittel - enthalten sein.

**Hautpflegemittel,** wie Cremes, Lotionen und dergleichen, aber auch Parfums, After Shaves, Tonics, Sprays und dekorative Kosmetikprodukte, weisen in der Regel - neben den bereits genannten Tensiden - einen Gehalt an Ölkörpern, Emulgatoren, Fetten und Wachsen, Stabilisatoren, Überfettungsmitteln, Verdickungsmitteln, biogenen Wirkstoffen, Filmbildnern, Konservierungsmitteln, Farb- und Duftstoffen auf.

**Haarpflegemittel**, wie beispielsweise Haarshampoos, Haarlotionen, Haarsprays, Schaumbäder und dergleichen, können als weitere Hilfs- und Zusatzstoffe - neben mit den Biopolymeren kompatiblen Tensiden - Emulgatoren, Überfettungsmittel, Verdickungsmittel, biogene Wirkstoffe, Filmbildner, Kationpolymere, Silicone Konservierungsmittel, Farb- und Duftstoffe enthalten.

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₀-Fettsäuren mit linearen C₆-C₂₀-Fettalkoholen, Ester von verzweigten C₆-C₁₃-Carbonsäuren mit linearen C₆-C₂₀-Fettalkoholen, Ester von linearen C₆-C₁₈-Fettsäuren mit verzweigten Alkoholen, insbesondere 2-Ethylhexanol, Ester von linearen und/oder verzweigten Fettsäuren mit mehr-wertigen Alkoholen (wie z.B. Dimerdiol oder Trimerdiol) und/oder Guerbetalkoholen, Trigly- ceride auf Basis C₆-C₁₀-Fettsäuren, pflanzliche Öle, verzweigte primäre Alkohole, substi-tuierte Cyclohexane, Guerbetcarbonate, Dialkylether und/oder aliphatische bzw. naphthe-nische Kohlenwasserstoffe in Betracht.

Als **Emulgatoren bzw. Co-Emulgatoren** können nichtionogene, ampholytische und/ oder zwitterionische grenzflächenaktive Verbindungen verwendet werden, die sich durch eine lipophile, bevorzugt lineare, Alkyl- oder Alkenylgruppe und mindestens eine hydrophile Gruppe auszeichnen. Diese hydrophile Gruppe kann sowohl eine ionogene als auch eine nichtionogene Gruppe sein.

Nichtionogene Emulgatoren enthalten als hydrophile Gruppe z. B. eine Polyolgruppe, eine Polyalkylenglycolethergruppe oder eine Kombination aus Polyol- und Polyglycolethergruppe. Bevorzugt sind solche Mittel, die als **O/W-Emulgatoren** nichtionogene Tenside aus mindestens einer der folgenden Gruppen enthalten: (al) Anla-gerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe; (a2) C_{12/18}-Fettsäuremono- und -diester von Anlagerungsprodukten von 1 bis 30 Mol Ethylenoxid an Glycerin; (a3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte; (a4) Alkylmono- und -oligoglycoside mit 8 bis 22 Kohlenstoffatomen im Alkylrest und deren ethoxylierte Analoga und (a5) Anlagerungsprodukte von 15 bis 60 Mol Ethylenoxid an Ricinusöl und/oder gehärtetes Ricinusöl; (a6) Polyol- und insbesondere Polyglycerinester wie z.B. Polyglycerinpolyricinoleat oder Polyglycerinpoly-12-hydroxystearat. Ebenfalls geeignet sind Gemische von Verbindungen aus mehreren dieser Substanzklassen. Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 20 24 051** als Rückfettungsmittel für kosmetische Zubereitungen bekannt. C_{8/18}-Alkyl-mono- und oligoglycoside, ihre Herstellung und ihre Verwendung als oberflächen-aktive Stoffe sind beispielsweise aus **US 3,839,318, US 3,707,535, US 3,547,828, DE-OS 19 43 689, DE-OS 20 36 472** und **DE-A1 30 01 064** sowie **EP-A 0 077 167** bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt. Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyl-dimethylammoniumglycinat, N-Acyl-aminopropyl-N,N-dimethylammoniumglycinate, beispielsweise das Kokosacylaminopropyldimethylammoniumglycinat, und 2-Alkyl-3-carboxyl- methyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung Cocamidopropyl Betaine bekannte Fett-säureamid-Derivat.

Ebenfalls geeignete Emulgatoren sind ampholytische Tenside. Unter ampholytischen Tensiden werden solche oberflächenaktiven Verbindungen verstanden, die außer einer C_{8/18}-Alkyl- oder - Acylgruppe im Molekül mindestens eine freie Aminogruppe und mindestens eine -COOH- oder -SO₂H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxy-ethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin.

Als **W/O-Emulgatoren** kommen in Betracht: (b1) Anlagerungsprodukte von 2 bis 15 Mol Ethylenoxid an Ricinusöl und/oder gehärtetes Ricinusöl; (b2) Partialester auf Basis linearer, verzweigter, ungesättigter bzw. gesättigter C_{12/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit) sowie Polyglucoside (z.B. Cellulose); (b3) Trialkylphosphate; (b4) Wollwachsalkohole; (b5) Polysiloxan-Polyalkyl-Polyether-Copolymere bzw. entsprechende Derivate; (b6) Mischester aus Pentaerythrit, Fettsäuren, Citronensäure und Fettalkohol gemäß DE-PS 11 65 574 sowie (b7) Polyalkylenglycole.

Geeignete **kationische Polymere** sind beispielsweise kationischen Cellulosederivate, kationischen Stärke, Copolymere von Diallylammoniumsalzen und Acrylamiden, quaternierte Vinylpyrrolidon/Vinylimidazol-Polymere wie z.B. Luviquat® (BASF AG, Ludwigshafen/ FRG), Kondensationsprodukte von Polyglycolen und Aminen, quaternierte Kollagenpolypeptide wie beispielsweise Lauryldimonium hydroxypropyl hydrolyzed collagen (Lamequat®L, Grünau GmbH), Polyethylenimin, kationische Siliconpolymere wie z.B. Amidomethicone oder Dow Corning, Dow Corning Co./US, Copolymere der Adipinsäure und Dimethylaminohydroxypropyldiethylentrimamin (Cartaretine®, Sandoz/CH), Polyaminopolyamide wie z.B. beschrieben in der FR-A 22 52 840 sowie deren vernetzte wasserlöslichen Polymere, kationische Chitinderivate wie beispielsweise quaterniertes Chitosan, gegebenen-falls mikrokristallin verteilt, kationischer Guar-Gum wie z.B. Jaguar® CBS, Jaguar® C-17, Jaguar® C-16 der Celanese/US, quaternierte Ammoniumsalz-Polymere wie z.B. Mirapol® A15, Mirapol® AD-1, Mirapol® AZ-1 der Miranol/US.

fluor- und/oder alkylmodifizierte Siliconverbindungen. Unter **biogenen Wirkstoffen** sind beispielsweise Pflanzenextrakte und Vitaminkomplexe zu verstehen. Gebräuchliche **Filmbildner** sind beispielsweise Polyvinylpyrrolidon, Vinylpyrrolidon-Vinylacetat-Copolymerisate, Polymere der Acrylsäurereihe, quaternäre Cellulose-Derivate, Kollagen, Hyaluron-säure bzw. deren Salze und ähnliche Verbindungen. Als **Überfettungsmittel** können Substanzen wie beispielsweise polyethoxylierte Lanolinderivate, Lecithinderivate, Polyolfettsäureester, Monoglyceride und Fettsäurealkanolamide verwendet werden, wobei die letz-teren gleichzeitig als Schaumstabilisatoren dienen. Typische Beispiele für **Fette** sind Glyce-ride, als **Wachse** kommen u.a. Bienenwachs, Paraffinwachs oder Mikrowachse gegebenen-falls in Kombination mit hydrophilen Wachsen, z.B. Cetylstearylalkohol in Frage. Als **Stabilisatoren** können Metallsalze von Fettsäuren wie z.B. Magnesium-, Aluminium und/oder Zinkstearat eingesetzt werden. Unter **biogenen Wirkstoffen** sind beispielsweise Pflanzenextrakte und Vitaminkomplexe zu verstehen. Als **Konservierungsmittel** eignen sich beispiels-weise Phenoxyethanol, Formaldehydlösung, Parabene, Pentandiol oder Sorbinsäure. Als Perlglanzmittel kommen beispielsweise Glycoldistearinsäureester wie Ethylenglycoldistea-rat, aber auch Fettsäuremonoglycolester in Betracht. Als **Farbstoffe** können die für kosmetische Zwecke geeigneten und zugelassenen Substanzen verwendet werden, wie sie beispielsweise in der Publikation "**Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-%-bezogen auf die Mittel - betragen. Die Herstellung der Mittel kann in an sich bekannter Weise, d.h. beispielsweise durch Heiß-, Kalt-, Heiß-Heiß/Kalt- bzw. PIT-Emulgierung erfol-gen. Hierbei handelt es sich um ein rein mechanisches Verfahren, eine chemische Reaktion findet nicht statt.

Die folgenden Beispiele sollen den Gegenstand der Erfindung näher erläutern, ohne ihn darauf einzuschränken.

### Beispiele

### I. Herstellbeispiele

### Beispiel 1:

1000 g frisch ausgelöste Krabbenschalen (Aschegehalt 31,8 Gew.-% bezogen auf das Trockengewicht) wurden getrocknet und über einen Zeitraum von 12 h bei 18°C mit 3000 ml verdünnter, 1 molarer Salzsäure bei einem pH-Wert von 0,5 behandelt. Anschließend wurde das demineralisierte Produkt neutral gewaschen; der Aschegehalt - bezogen auf Trockengewicht - betrug 0,79 Gew.-%. Das demineralisierte Produkt aus (a) wurden mit 15 Gew.-%iger Natriumhydroxidlösung im Gewichtsverhältnis 1 : 3 versetzt und 2 h bei 70°C gerührt. Anschließend wurde das deproteinierte Produkt 18 h bei 30°C gehalten. Das resultierende Material wurde wieder neutral gewaschen; der Aschegehalt - bezogen auf Trockengewicht - betrug 0,6 Gew.-%. Das deproteinierte Produkt wurde wiederum im Gewichtsverhältnis 1 : 3 mit 1 molarer Salzsäure versetzt. Der Ansatz wurde bei 18°C etwa 1,5 h leicht gerührt und anschließend neutral gewaschen. Das decalcifizierte, leicht rosa gefärbte Produkt wies einen Aschegehalt - bezogen auf Trockengewicht - von 0,08 Gew.-% auf und wurde vor der Weiterverarbeitung durch Abpressen weitgehend von anhaftendem Wasser befreit. Das decalcifizierte Produkt wurde im Gewichtsverhältnis 1 : 4 in kochender Natronlauge (70 Gew.-%ig) gelöst und dort 4 h bei 108°C gehalten. Danach wurde das resultierende Biopolymer mit Wasser neutral gewaschen, gefriergetrocknet und zermahlen. Es wurde ein blaß rosa gefärbtes Pulver erhalten. Die Ergebnisse sind in Tabelle 1 zusammengefaßt. Die Molekulargewichte wurden via HPLC, die Deacetylierungsgrade via ¹H-NMR-Spektroskopie ermittelt.

### Beispiel 2

Beispiel 1 wurde wiederholt, die Deacetylierung jedoch über einen Zeitraum von 2 h durchgeführt. Die Ergebnisse sind in Tabelle 1 zusammengefaßt.

### Beispiel 3

Beispiel 1 wurde wiederholt, jedoch unter Verzicht der Zwischentrocknung. Die Ergebnisse sind in Tabelle 1 zusammengefaßt.

### Vergleichsbeispiel V1

1000 g frische Krustentierschalen wurden 24 h bei Raumtemperatur mit 3 1 1N Salzsäure versetzt, neutral gewaschen, mit 5 Gew.-% 2N Natronlauge versetzt, 3 h bei 90°C gerührt und wieder neutral gewaschen. Das Vergleichsbeispiel entspricht dem Beispiel 1 der **FR-A 27 01 266.** Die Ergebnisse sind in Tabelle 1 zusammengefaßt.

### Vergleichsbeispiel V2

1000 g frische Krustentierschalen wurden 5 h bei Raumtemperatur mit 3 1 2N Salzsäure versetzt, neutral gewaschen, mit 512N Natronlauge versetzt, 36 h bei 100°C gerührt, neutral gewaschen, 2 h bei Raumtemperatur mit 512N Salzsäure gerührt, neutral gewaschen, schließlich 1 h mit 111N Natronlauge bei Raumtemperatur nachbehandelt und wieder neutral gewaschen. Das Vergleichsbeispiel entspricht dem modifizierten Hackmann-Abbau gemäß der Schrift **Makromol. Chem. 177, 3589 (1976)**. Die Ergebnisse sind in Tabelle 1 zusammengefaßt.

### Vergleichsbeispiele V3 und V4

Hier wurden kationische Biopolymere der Fa. Ajinomoto/Japan, also Produkte des Marktes untersucht.

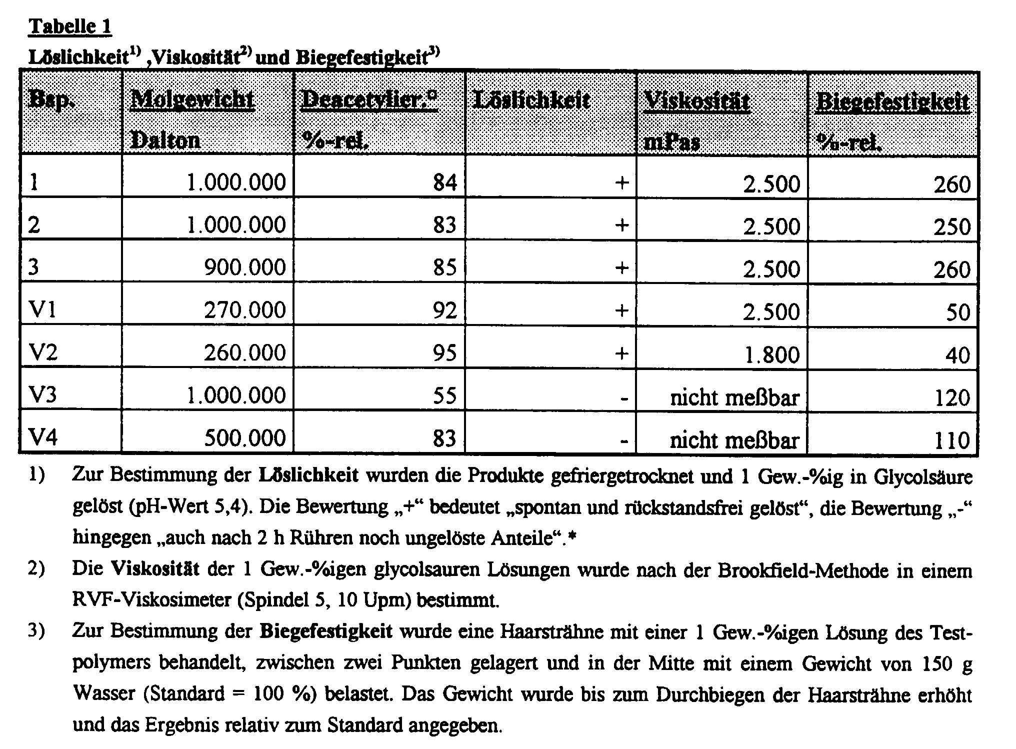

Die Beispiele und Vergleichsbeispiele zeigen, daß nur die erfindungsgemäßen Produkte, die ein ausgewähltes Molekulargewicht und einen vorteilhaften Deacetylierungsgrad besitzen, die Anforderungen an Viskosität, Löslichkeit und Biegefestigkeit gleichzeitig erfüllen.

### II. Rezepturbeispiele

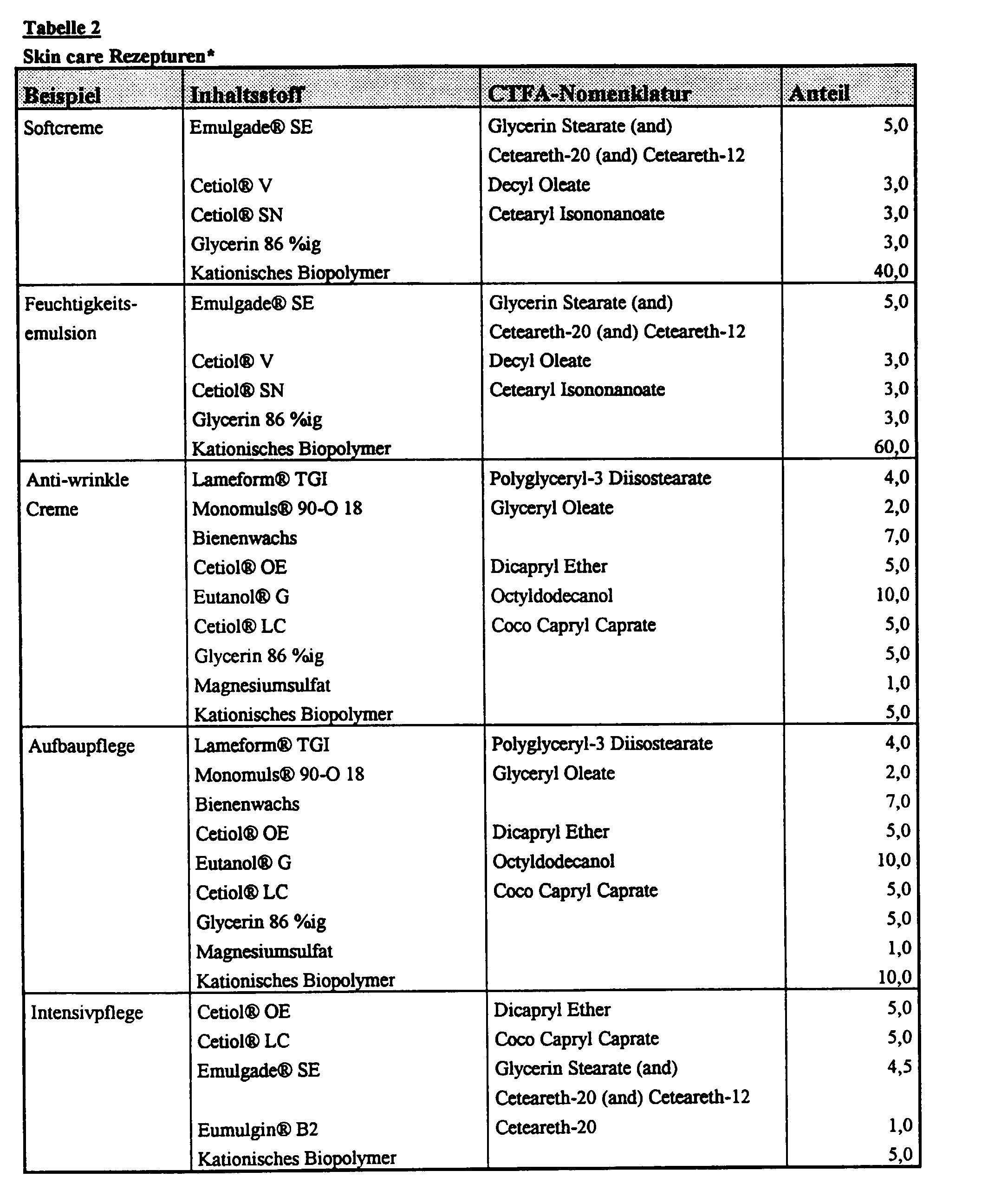

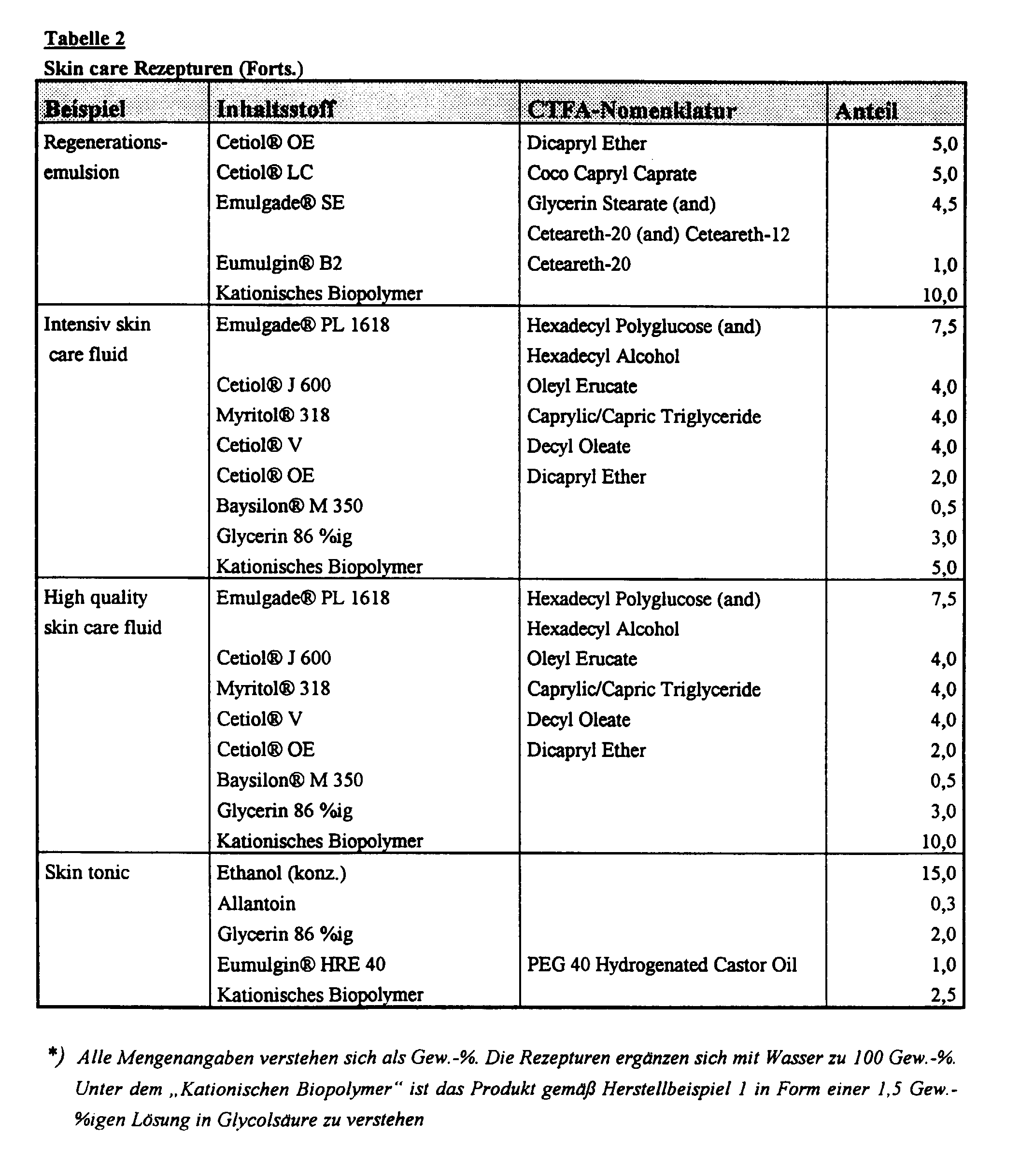

## Patentansprüche

1. Kationische Biopolymere mit einem durchschnittlichen Molekulargewicht im Bereich von 800.000 bis 1.200.000 Dalton gemäß PLC einer Viskosität nach Brookfield (1 Gew.-%ig in Glycolsäure) unterhalb von 5000 mPas, einem Deacetylierungsgrad gemäß ¹H-NMR im Bereich von 80 bis 88 % und einem Aschegehalt von weniger als 0,3 Gew.-%, dadurch erhältlich, daß man
(a) frische Krustentierschalen mit verdünnter wäßriger Mineralsäure behandelt,
(b) das resultierende demineralisierte erste Zwischenprodukt mit wäßriger Alkalihydroxidlösung behandelt,
(c) das resultierende geringfügig deproteinierte zweite Zwischenprodukt erneut mit verdünnter wäßriger Mineralsäure behandelt,
(d) gegebenenfalls das resultierende decalcifizierte dritte Zwischenprodukt bis auf einen Restwassergehalt von 5 bis 25 Gew.-% trocknet und
(e) schließlich mit konzentrierter wäßriger Alkalilauge deacetyliert,
wobei man die Schritte (a) und (c) bei einer Temperatur im Bereich von 15 bis 25°C und einem pH-Wert von 0,3 bis 0,7 und die Schritte (b) und (e) bei einer Temperatur im Bereich von 70 bis 110°C und einem pH-Wert von 12 bis 14 durchführt.

2. Verfahren zur Herstellung von kationischen Biopolymeren mit einem durchschnittlichen Molekulargewicht im Bereich von 800.000 bis 1.200.000 Dalton gemäß PLC einer Viskosität nach Brookfield (1 Gew.-%ig in Glycolsäure) unterhalb von 5000 mPas, einem Deacetylierungsgrad gemäß ¹H-NMR im Bereich von 80 bis 88 % und einem Aschegehalt von weniger als 0,3 Gew.-%, dadurch erhältlich, daß man
(a) frische Krustentierschalen mit verdünnter wäßriger Mineralsäure behandelt,
(b) das resultierende demineralisierte erste Zwischenprodukt mit wäßriger Alkalihydroxidlösung behandelt,
(c) das resultierende geringfügig deproteinierte zweite Zwischenprodukt erneut mit verdünnter wäßriger Mineralsäure behandelt,
(d) gegebenenfalls das resultierende decalcifizierte dritte Zwischenprodukt bis auf einen Restwassergehalt von 5 bis 25 Gew.-% trocknet und
(e) schließlich mit konzentrierter wäßriger Alkalilauge deacetyliert,
(e) schließlich mit konzentrierter wäßriger Alkafilauge deacetyliert,
wobei man die Schritte (a) und (c) bei einer Temperatur im Bereich von 15 bis 25°C und einem pH-Wert von 0,3 bis 0,7 und die Schritte (b) und (e) bei einer Temperatur im Bereich von 70 bis 110°C und einem pH-Wert von 12 bis 14 durchführt.

3. Verfahren nach Anspruch 2, **dadurch gekennzeichnet**, daß man als Mineralsäure wäßrige Salzsäure und als Alkalihydroxidlösung Natriumhydroxidlösung einsetzt.

4. Verwendung der kationischen Biopolymeren gemäß Anspruch 1 zur Herstellung von kosmetischen und/oder pharmazeutischen Mitteln.

## Claims

1. Cationic biopolymers with an average molecular weight of 800,000 to 1,200, 000 Dalton as determined by HPLC, a Brookfield viscosity (1% by weight in glycolic acid) below 5,000 mPas, a degree of deacetylation as determined by ¹H-NMR of 80 to 88% and an ash content of less than 0.3% by weight, obtainable by
(a) treating fresh crustacean shells with dilute aqueous mineral acid,
(b) treating the resulting demineralized first intermediate product with aqueous alkali metal hydroxide solution,
(c) treating the resulting lightly deproteinized second intermediate product with more dilute aqueous mineral acid,
(d) optionally drying the resulting decalcified third intermediate product to a residual water content of 5 to 25% by weight and
(d) finally deacetylating the optionally dried product with concentrated aqueous alkali metal hydroxide,
steps (a) and (c) being carried out at a temperature of 15 to 25°C and at a pH value of 0.3 to 0.7 and steps (b) and (e) being carried out at a temperature of 70 to 110°C and at a pH value of 12 to 14.

2. A process for the production of cationic biopolymers having an average molecular weight of 800,000 to 1,200, 000 Dalton as determined by HPLC, a Brookfield viscosity (1% by weight in glycolic acid) below 5,000 mPas, a degree of deacetylation as determined by ¹H-NMR of 80 to 88% and an ash content of less than 0.3% by weight, comprising the steps of
(a) treating fresh crustacean shells with dilute aqueous mineral acid,
(b) treating the resulting demineralized first intermediate product with aqueous alkali metal hydroxide solution,
(c) treating the resulting lightly deproteinized second intermediate product with dilute aqueous mineral acid,
(d) optionally drying the resulting decalcified third intermediate product to a residual water content of 5 to 25% by weight and
(d) finally treating the optionally dried product with concentrated aqueous alkali metal hydroxide,
steps (a) and (c) being carried out at a temperature of 15 to 25°C and at a pH value of 0.3 to 0.7 and steps (b) and (e) being carried out at a temperature of 70 to 110°C and at a pH value of 12 to 14.

3. A process as claimed in claim 2, c**haracterized in that** aqueous hydrochloric acid is used as the mineral acid and sodium hydroxide solution as the alkali metal hydroxide solution.

4. The use of the cationic biopolymers claimed in claim 1 for the production of cosmetic and/or pharmaceutical formulations.

## Revendications

1. Biopolymères cationiques ayant un poids moléculaire moyen dans la zone de 800 000 à 1 200 000 Dalton déterminé par HPLC, une viscosité selon Brookfield (à 1% en poids dans l'acide glycolique) en dessous de 5 000 mPas un degré de désacécylation déterminé selon [¹H-RMN] dans la zone de 80 à 88 % et une teneur en cendres de moins de 0,3 % en poids,
que l'on peut obtenir en ce qu' :
a) on traite des coquilles fraîches de crustacées avec des acides minéraux aqueux dilués,
b) on traite le premier produit intermédiaire déminéralisé résultant avec une solution aqueuse d'hydroxyde de métal alcalin,
c) on traite le second produit intermédiaire résultant faiblement déprotéiné, à nouveau avec des acides minéraux aqueux dilués,
d) on sèche le cas échéant le troisième produit intermédiaire résultant décalcifié jusqu'à une teneur résiduelle en eau allant de 5 à 25 % en poids, et
e) enfin on désacétyle avec une lessive alcaline aqueuse concentrée.
dans lequel on exécute les étapes a) et c) à une température dans la plage de 15 à 25°C et à une valeur de pH de 0,3 à 0,7 et les étapes b) et e) à une température dans la plage de 70 à 110°C et à une valeur de pH de 12 à 14.

2. Procédé de fabrication de biopolymères cationiques ayant un poids moléculaire moyen dans la zone de 800 000 à 1 200 000 Dalton déterminé par HPLC, une viscosité selon Brookfield (à 1 % en poids dans l'acide glycolique) en dessous de 5 000 mPas, un degré de désacétylation déterminé par spectre [¹H-RMN] dans la zone de 80 à 88 % et une teneur en cendres de moins de 0,3 % en poids,
que l'on peut obtenir en ce qu' :
a) on traite des coquilles fraîches de crustacées avec des acides minéraux aqueux dilués,
b) on traite le premier produit intermédiaire déminéralisé résultant avec une solution aqueuse d'hydroxyde de métal alcalin,
c) on traite le second produit intermédiaire résultant faiblement déprotéiné, à nouveau avec des acides minéraux aqueux dilués,
d) on sèche le cas échéant le troisième produit intermédiaire résultant décalcifié jusqu'à une teneur résiduelle en eau allant de 5 à 25 % en poids, et
e) enfin on désacétyle avec une lessive alcaline aqueuse concentrée.
dans lequel on exécute les étapes a) et c) à une température dans la plage de 15 à 25°C et à une valeur de pH de C,3 à 0,7 et les étapes b) et e) à une température dans la plage de 70 à 110°C et à une valeur de pH de 12 à 14.

3. Procédé selon la revendication 2,
caractérisé en ce qu'
on met en oeuvre comme acide minéral, un acide chlorhydrique aqueux et comme solution d'hydroxyde de métal alcalin, une solution d'hydroxyde de sodium.

4. Utilisation des biopolymères cationiques conformément à la revendication 1, pour l'obtention de compositions cosmétiques et/ou pharmaceutiques.
